# EUROPEAN PATENT SPECIFICATION

(11) **EP 3 826 642 B1**
(45) Date of publication and mention of the grant of the patent: **07.05.2025**
(21) Application number: 19742218.1
(22) Date of filing: 23.07.2019
(51) Int. Cl.: A61K 31/7048, A61K 31/55, A61K 45/06, A61P 13/12, A61P 43/00, A61K 9/20

(54) **EMPAGLIFLOZIN FOR USE IN TREATING ALPORT SYNDROME**
EMPAGLIFLOZIN ZUR BEHANDLUNG DES ALPORT-SYNDROMS
EMPAGLIFLOZINE POUR SON UTILISATION DANS LE TRAITEMENT DU SYNDROME D'ALPORT

(30) Priority: 25.07.2018 EP 18185598
(43) Date of publication of application: 02.06.2021
(73) Proprietor: Boehringer Ingelheim International GmbH, 55216 Ingelheim am Rhein (DE)
(72) Inventor: VON EYNATTEN, Maximilian, 55216 INGELHEIM AM RHEIN (DE); GROSS, Oliver, 37085 GOETTINGEN (DE); HAUSKE, Sibylle, Jenny, 55216 INGELHEIM AM RHEIN (DE)
(74) Representative: Hoffmann Eitle
(86) International application number: PCT/EP2019/069815
(87) International publication number: WO 2020/020896

(56) References cited:
- WO-A1-2014/028059
- WO-A1-2016/014781
- WO-A2-2016/029027
- ANONYMOUS: "NOTE FOR GUIDANCE ON THE EVALUATION OF THE PHARMACOKINETICS OF MEDICINAL PRODUCTS IN PATIENTS WITH IMPAIRED RENAL FUNCTION DISCUSSION", CHMP/EWP/225/02, 23 June 2004 (2004-06-23), pages 1 - 11, Retrieved from the Internet <URL:http://www.ema.europa.eu/docs/en_GB/document_library/Scientific_guideline/2009/09/WC500003123.pdf> [retrieved on 20170608]
- BOECKHAUS JAN ET AL: "MO1034: Sodium-Glucose Cotransporter 2 Inhibitors in Patients with Alport Syndrome and Fsgs: A Case Series", NEPHROLOGY DIALYSIS TRANSPLANTATION, vol. 37, no. Supplement_3, 3 May 2022 (2022-05-03), GB, ISSN: 0931-0509, DOI: 10.1093/ndt/gfac089.011
- GE MENGYUAN ET AL: "Empagliflozin reduces podocyte lipotoxicity in experimental Alport syndrome", ELIFE, vol. 12, 2 May 2023 (2023-05-02), GB, pages e83353, ISSN: 2050-084X, DOI: 10.7554/eLife.83353
- BOECKHAUS JAN ET AL: "MO1034: Sodium-Glucose Cotransporter 2 Inhibitors in Patients with Alport Syndrome and Fsgs: A Case Series", NEPHROLOGY DIALYSIS TRANSPLANTATION, vol. 37, no. Supplement_3, 3 May 2022 (2022-05-03), GB, ISSN: 0931-0509, DOI: 10.1093/ndt/gfac089.011
- GE MENGYUAN ET AL: "Empagliflozin reduces podocyte lipotoxicity in experimental Alport syndrome", ELIFE, vol. 12, 2 May 2023 (2023-05-02), GB, pages e83353, ISSN: 2050-084X, DOI: 10.7554/eLife.83353
- CHRISTOPH WANNER ET AL: "Empagliflozin and Progression of Kidney Disease in Type 2 Diabetes", THE NEW ENGLAND JOURNAL OF MEDICINE, - NEJM -, vol. 375, no. 4, 28 July 2016 (2016-07-28), US, pages 323 - 334, XP055442115, ISSN: 0028-4793, DOI: 10.1056/NEJMoa1515920
- ROSER TORRA ET AL: "Abstract", NEPHROLOGY DIALYSIS TRANSPLANTATION., vol. 34, no. 8, 13 June 2019 (2019-06-13), GB, pages 1272 - 1279, XP055621058, ISSN: 0931-0509, DOI: 10.1093/ndt/gfz131
- USHA PANCHAPAKESAN ET AL: "Effects of SGLT2 Inhibition in Human Kidney Proximal Tubular Cells-Renoprotection in Diabetic Nephropathy?", PLOS ONE, vol. 8, no. 2, 4 February 2013 (2013-02-04), pages e54442, XP055119255, ISSN: 1932-6203, DOI: 10.1371/journal.pone.0054442

## Description

### Technical Field of the Invention

The present invention relates to empagliflozin for use in methods for treating Alport syndrome, various aspects and symptoms associated with Alport syndrome. In addition the present invention relates to pharmaceutical compositions comprising empagliflozin, in particular for use in methods as described hereinbefore and hereinafter.

### Background of the Invention

Alport syndrome (AS), also called hereditary nephritis, is a genetically heterogeneous disease characterized by haematuric glomerulopathy variably associated with renal failure, hearing loss, anterior lenticonus and retinal flecks. Furthermore Alport Syndrome is characterized by a lamellated glomerular basement membrane (GBM) with an abnormal collagen IV composition. It is described as being caused by mutations that impair the production, deposition, or function of the collagen IV alpha345 network, the major collagenous constituent of mature basement membranes in the glomerulus, cochlea, cornea, lens, and retina. The X-linked form of Alport syndrome results from mutations in COL4A5, which encodes the collagen IV alpha5 chain. Autosomal forms of Alport syndrome are caused by mutations in COL4A3 and COL4A4, which are located on chromosome 2 and encode the collagen IV alpha3 and alpha4 chains, respectively. Mutations in both alleles of COL4A3 or COL4A4 are associated with autosomal recessive transmission, while heterozygous mutations cause autosomal dominant disease. The diagnosis of Alport Syndrome is suspected when an individual has glomerular hematuria or renal failure and a family history of Alport syndrome or renal failure without other obvious cause. The diagnosis of Alport syndrome is highly likely if there are glomerular hematuria and a family history of Alport syndrome with not other cause of hematuria; if bilateral high-tone sensorineural hearing loss, lenticonus, or fleck retinopathy is present; or if the GBM lacks the collagen IV alpha5 chain. [O. Gross et al., Nephrol. Dial. Transplant (2017) 32: 916-924; J. Savige et al., J. Am. Soc. Nephrol. 24: 364-375, 2013; C. Kashtan, F1000Research 2017, 6 (F1000 Faculty Rev):50]. Proteinuria, hearing loss, lenticonus, retinopathy, and reduced levels of GBM collagen IV a5 chain all correlate with an increased likelihood of early-onset renal failure in males. Angiotensin-converting enzyme (ACE) inhibitors are administered to reduce proteinuria. Angiotensin-receptor blockers and aldosterone inhibitors have additional benefits for proteinuria. [J. Savige et al., dto.]. About 10,000 children and about 25,000 adults are diagnosed as having Alport syndrome in Europe and the US. Stages of the Alport syndrome are from hematuria (bloody urine), microalbuminuria, proteinuria, declining glomerular filtration rate (GFR) to end-stage kidney disease (ESKD) with start of dialysis at an age of about 22 to 45 years. To date there is no curative treatment for Alport syndrome and despite interventions with RAS blockers, most patients with Alport syndrome will continue to face progressive loss in kidney function and remain at high risk for ESKD. Thus, there is a high unmet medical need to explore potential novel treatment options for patients with Alport syndrome, including those already receiving current standard of care and remaining at high ESKD risk.

WO 2016/014781 discloses collagen replacement therapy for use in the treatment of Alport syndrome. WO 2016/029027 discloses the peptide D-Arg-2',6'-Dmt-Lys-Phe-NH₂ for use in the treatment of Alport syndrome. WO 2014/028059 discloses an RAC1 inhibitor and/or a CDC42 inhibitor for use in the treatment of Alport syndrome.

### Summary of the Invention

The present invention relates to empagliflozin for use in treating Alport syndrome in a patient in need thereof. The invention is set out in the appended set of claims.

In another aspect the present invention relates to empagliflozin for use in delaying progression of chronic kidney disease in a patient diagnosed with Alport syndrome.

In yet another aspect the present invention relates to empagliflozin for use in treating or alleviating glomerular hypertension in a patient diagnosed with Alport syndrome.

In yet another aspect the present invention relates to empagliflozin for use in treating, including slowing or reversing the progression of albuminuria, including microalbuminuria and macroalbuminuria, in a patient diagnosed with Alport syndrome.

In yet another aspect the present invention relates to empagliflozin for use in treating, including slowing or reversing the progression of proteinuria in a patient diagnosed with Alport syndrome.

The present invention further provides for a pharmaceutical composition comprising empagliflozin for use in any one of the methods described herein.

In the methods and uses according to this invention empagliflozin is optionally administered in combination with one or more other therapeutic agents to the patient.

The pharmaceutical composition comprising empagliflozin for use according to this invention may comprise one or more other therapeutic agents.

Further aspects of the present invention become apparent to the one skilled in the art by the description hereinbefore and in the following and by the examples.

### Definitions

The term **"active ingredient"** of a pharmaceutical composition according to the present invention means the SGLT2 inhibitor empagliflozin according to the present invention. An "active ingredient" is also sometimes referred to herein as an "active substance".

The term **"empagliflozin"** refers to the SGLT2 inhibitor 1-chloro-4-(β-D-glucopyranos-1-yl)-2-[4-((S)-tetrahydrofuran-3-yloxy)-benzyl]-benzene of the formula 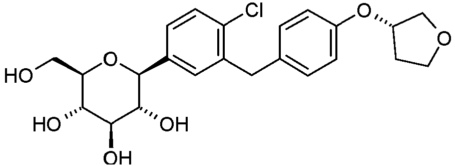 as described for example in WO 2005/092877. Methods of synthesis are described in the literature, for example WO 06/120208 and WO 2011/039108. According to this invention, it is to be understood that the definition of empagliflozin also comprises its hydrates, solvates and polymorphic forms thereof, and prodrugs thereof. An advantageous crystalline form of empagliflozin is described in WO 2006/117359 and WO 2011/039107. This crystalline form possesses good solubility properties which enables a good bioavailability of the SGLT2 inhibitor. Furthermore, the crystalline form is physico-chemically stable and thus provides a good shelf-life stability of the pharmaceutical composition. Preferred pharmaceutical compositions, such as solid formulations for oral administration, for example tablets, are described in WO 2010/092126.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating",** "preventivally treating" and "preventing" are used interchangeably and comprise a treatment of patients at risk to develop a condition mentioned hereinbefore, thus reducing said risk.

The term **"tablet"** comprises tablets without a coating and tablets with one or more coatings. Furthermore the "term" tablet comprises tablets having one, two, three or even more layers and press-coated tablets, wherein each of the beforementioned types of tablets may be without or with one or more coatings. The term "tablet" also comprises mini, melt, chewable, effervescent and orally disintegrating tablets.

The terms **"pharmacopoe"** and **"pharmacopoeias"** refer to standard pharmacopoeias such as the "USP 31-NF 26 through Second Supplement" (United States Pharmacopeial Convention) or the "European Pharmacopoeia 6.3" (European Directorate for the Quality of Medicines and Health Care, 2000-2009).

The term **"Alport syndrome"** is characterized by one or more of the following conditions as can be found in the patient:
a) hematuria or hematuric glomerulopathy variably associated with renal failure, hearing loss, anterior lenticonus and retinal flecks;
b) a lamellated glomerular basement membrane (GBM) with an abnormal collagen IV composition;
c) one or more mutations that impair the production, deposition, or function of the collagen IV alpha345 network;
d) one or more mutations in COL4A5, which encodes the collagen IV alpha5 chain;
e) one or more mutations in COL4A3, which encodes the collagen IV alpha3 chain;
f) one or more mutations in COL4A4, which encodes the collagen IV alpha4 chain;
g) one or more mutations in COL4A3 and COL4A4, which encode the collagen IV alpha3 and alpha4 chains, respectively.

The Alport syndrome may be X-linked, autosomal dominant or autosomal recessive.

The term **"hematuria"** is defined as a condition characterized by the presence of red blood cells in the urine.

The term **"hematuric glomerulopathy"** is defined as a condition characterized by the presence of red blood cells in the urine caused by affected glomeruli of the nephron.

The term **"proteinuria"** is defined as a condition wherein there is a presence of excess proteins in the urine. The protein content in urine is usually measured as a concentration in mg/dL. The concentration of protein in the urine may be compared to the creatinine level in the urine sample. This is termed the protein/creatinine ratio (PCR). Usually proteinuria is defined as a protein/creatinine ratio (PCR) greater than 45 mg/mmol, which is equivalent to an albumin-to-creatinine ration (ACR) of greater than 30 mg/mmol or approximately 300 mg/g.

The term **"albuminuria"** is defined as a condition wherein more than the normal amount of albumin is present in the urine. Albuminuria can be determined by the albumin excretion rate (AER) and/or the albumin-to-creatine ratio (ACR) in the urine (also refered to as UACR). Albuminuria categories in CKD are defined as follows:

| | | ACR (approximate equivalent) | | |
|---|---|---|---|---|
| Category | AER (mg/24 hours) | (mg/mmol) | (mg/g) | Terms |
| A1 | < 30 | < 3 | < 30 | Normal to mildly increased |
| A2 | 30-300 | 3-30 | 30-300 | Moderately increased |
| A3 | >300 | >30 | >300 | Severely increased |

Category A1 reflects no albuminuria, category A2 reflects microalbuminuria, category A3 reflects macroalbuminuria. The progression of category A1 usually leads to microalbuminuria (A2) but may also directly result in macroalbuminuria (A3). Progression of microalbuminuria (A2) results in macroalbuminuria (A3).

The term **"eGFR"** refers to the estimated glomerular filtration rate (GFR). The GFR describes the flow rate of filtered fluid through the kidney. The estimated GFR may be calculated based on serum creatinine values e.g. using the Chronic Kidney Disease Epidemiology Collaboration (CKD-EPI) equation, the Cockcroft-Gault formula or the Modification of Diet in Renal Disease (MDRD) formula, which are all known in the art. According to an aspect of this invention the estimated glomerular filtration rate (eGFR) is derived from serum creatinine values, age, sex and race based on the CKD-EPI equation: GFR = 141 × min (Scr/κ, 1)α × max(Scr /κ, 1)-1.209 × 0.993Age × 1.018 [if female] × 1.159 [if black] where:
Scr is serum creatinine in mg/dL,
κ is 0.7 for females and 0.9 for males,
α is -0.329 for females and -0.411 for males,
min indicates the minimum of S_{cr} /κ or 1, and
max indicates the maximum of S_{cr} /κ or 1.

For the purpose of the present invention, the degree of renal impairment in a patient is defined by the following estimated glomerular filtration rate (eGFR):
Normal renal function (CKD stage 1): eGFR ≥ 90 mL/min/1.73 m²
Mild renal impairment (CKD stage 2): eGFR ≥60 to <90 mL/min/1.73 m²
Moderate renal impairment (CKD stage 3): eGFR ≥30 to <60 mL/min/1.73 m²
Severe renal impairment (CKD stage 4): eGFR ≥15 to <30 mL/min/1.73 m²
Kidney failure (CKD stage 5): eGFR <15 mL/min/1.73 m²

According to the present invention moderate renal impairment can be further divided into two sub-stages:
Moderate A renal impairment (CKD 3A): eGFR ≥45 to <60 mL/min/1.73 m²
Moderate B renal impairment (CKD 3B): eGFR ≥30 to <45 mL/min/1.73 m²

The term "chronic kidney disease (CKD)" is defined as abnormalities of kidney structure or function, in particular present for more than three months, with implications for health. CKD is classified based on cause, GFR category, and albuminuria category (CGA).

CKD has been classified into 5 stages, where stage 1 is kidney damage with normal GFR (mL/min/1.73 m2 ) of 90 or above; stage 2 is kidney damage with a mild decrease in GFR (GFR 60-89); stage 3 is a moderate decrease in GFR (GFR 30-59); stage 4 is a severe decrease in GFR (GFR 15-29); and stage 5 is kidney failure (GFR <15 or dialysis). Stage 3 has been subdivided into stage 3A, which is a mild to moderate decrease in GFR (GFR 45-59), and stage 3B, which is a moderate to severe decrease in GFR (GFR 30-44).

### Detailed Description of the Invention

Beyond an improvement of glycemic control and weight loss due to an increase in urinary glucose excretion, empagliflozin shows a diuretic effect, reduced arterial stiffness and direct vascular effects (Cherney et al., Cardiovasc Diabetol. 2014;13:28; Cherney et al., Circulation. 2014;129:587-597). In the EMPA-REG OUTCOME^{™} study it was demonstrated that empagliflozin reduced the risk of cardiovascular death, hospitalization for heart failure and overall mortality in patients with type 2 diabetes mellitus and high cardiovascular risk (Zinman et al., N Engl J Med. 2015;373:2117-2128). It was observed that treatment with empagliflozin leads to blood pressure reductions without clinically relevant changes of the heart rate, thus improving rate pressure product (RPP), a surrogate marker of cardiac oxygen demand. Furthermore empagliflozin was found of not being associated with clinically relevant reflex-mediated sympathetic activation in contrast to increases observed with diuretics. It may be assumed that altered glucose and sodium gradients within the kidney may generate a sympathoinhibitory afferent renal nerve signal. The lack of sympathetic activation may contribute to a beneficial cardiovascular and renal profile of empagliflozin (cardiorenal axis). In secondary outcomes analyses of the EMPA-REG OUTCOME^{™} trial in patients with type 2 diabetes and established cardiovascular disease empagliflozin slowed progression of kidney disease and reduced the risk for clinically relevant kidney events (inclusive end-stage kidney disease). Kidney effects of empagliflozin were consistent in patients with or without prevalent chronic kidney disease (CKD) at baseline and overall results were observed in a population of patients whose blood pressure was well managed, with use of RAS blockers.

The underlying pathophysiology for progressive renal injury may vary among different CKD entities; however, considerable experimental and clinical evidence exists that at more advanced stages of CKD (i.e. in individuals with either significant loss of GFR and/or high levels of proteinuria, conditions also frequently seen in patients with Alport syndrome) a common tubular dysregulation may drive overall progression of kidney disease. This tubular dysregulation is characterized by hyper-reabsorption of sodium, leading to glomerular hypertension and renal barotrauma, a condition of particular importance for patients with Alport syndrome due to the frail and immature glomerular basement membrane (GBM). Glomerular hypertension is assumed to be the key culprit in progression of kidney disease, but effective treatments beyond RAS blockade are lacking. Empagliflozin has the potential to alleviate glomerular hypertension via blocking the sodium-glucose co-transporter 2 (SGLT2) in the proximal tubules in patients with Alport syndrome, in particular those at high risk of progressive kidney function loss. Lowering glomerular hypertension empagliflozin is expected to alleviate the stress and damage in nephrons of patients with Alport syndrome. The use of empagliflozin in the treatment of Alport syndrome is described hereinbefore and hereinafter.

The present invention relates to empagliflozin for use in treating Alport syndrome in a patient in need thereof. The term treating Alport syndrome includes delaying the occurrence or slowing or reversing the progression of Alport syndrome. In addition the term treating Alport syndrome includes treating or preventing the signs or symptoms of Alport syndrome. The term "treating Alport syndrome", including the signs or symptoms thereof, particularly refers to the kidney related manifestations, signs or symptoms, including those which are caused or mediated by a decline or loss of kidney function.

According to an aspect of the present invention the signs of symptoms of Alport syndrome include one or more of hematuria, proteinuria, cylindruria, leukocyturia, hypertension, edema, microalbuminuria, macroalbuminuria, overt albuminuria, declining glomerular filtration rate, interstitial fibrosis, interstitial inflammation, tubular damage, GBM ultrastructural abnormalities, nephrotic syndrome, glomerulonephritis, end-stage kidney disease, chronic anemia, macrothrombocytopenia, osteodystrophy, sensorineural deafness, anterior lenticonus, dot-and-fleck retinopathy, posterior polymorphous corneal dystrophy, recurrent corneal erosion, temporal macular thinning, cataracts, lacrimation, photophobia, vision loss, keratoconus, and leiomyomatosis, in particular proteinuria, hypertension, edema, microalbuminuria, macroalbuminuria, overt albuminuria, declining glomerular filtration rate, nephrotic syndrome, glomerulonephritis, end-stage kidney disease.

According to an aspect of the present disclosure there is provided a method for improving life expectancy in a patient diagnosed with Alport syndrome comprising administering empagliflozin to the patient.

In one embodiment, the present invention relates to empagliflozin for use in treating, preventing, protecting against, delaying the occurrence of or reducing the risk of one or more of:
- loss in eGFR, for example annual loss in eGFR,
- albuminuria,
- proteinuria,
- new onset of albuminuria,
- end-stage kidney disease (ESKD),
- progression from no albuminuria to micro- or macroalbuminuria,
- progression from microalbuminuria to macroalbuminuria,
- progression from macroalbuminuria to end-stage kidney disease (ESKD),
- doubling of serum creatinine level accompanied by an eGFR (based on modification of diet in renal disease (MDRD) formula) ≤45 mL/min/1.73m²,
- sustained reduction of ≥30%, ≥40%, ≥50% or ≥57% eGFR (CKD-EPI), in particular sustained reduction of ≥40% eGFR (CKD-EPI),
- sustained eGFR (CKD-EPI) <15 mL/min/1.73 m² for patients with baseline eGFR ≥30 mL/min/1.73 m²,
- sustained eGFR (CKD-EPI) <10 mL/min/1.73 m² for patients with baseline eGFR <30 mL/min/1.73 m²,
- need for continuous renal replacement therapy,
- need for dialysis treatment, in particular chronic dialysis treatment,
- need for receiving a renal transplant,
- death due to renal failure,
in a patient diagnosed with Alport syndrome.

According to an aspect of the invention the patient with Alport syndrome has prevalent chronic kidney disease.

According to this aspect of the invention the patient may have a decreased renal function according to one or more of the following groups
a) mild renal impairment, i.e. a glomerular filtration rate (GFR or eGFR) from 60 to 89 mL/min/1.73 m²,
b) moderate renal impairment, i.e. a glomerular filtration rate (GFR or eGFR) from 30 to 59 mL/min/1.73 m²,
c) moderate A renal impairment, i.e. a glomerular filtration rate (GFR or eGFR) from 45 to 59 mL/min/1.73 m²,
d) moderate B renal impairment, i.e. a glomerular filtration rate (GFR or eGFR) from 30 to 44 mL/min/1.73 m²,
e) severe renal impairment, i.e. a glomerular filtration rate (GFR or eGFR) from 15 to 29 mL/min/1.73 m²,
f) mild to severe renal impairment, i.e. a glomerular filtration rate (GFR or eGFR) from 15 to 89 mL/min/1.73 m²,
g) moderate to severe renal impairment, i.e. a glomerular filtration rate (GFR or eGFR) from 15 to 59 mL/min/1.73 m²,
h) mild to moderate renal impairment, i.e. a glomerular filtration rate (GFR or eGFR) from 30 to 89 mL/min/1.73 m²,
i) a glomerular filtration rate (GFR or eGFR) from 20 to 74 mL/min/1.73 m².

According to an aspect of the invention the patient has a decreased renal function with
a glomerular filtration rate (GFR or eGFR) below 90 mL/min/1.73 m², for example determined via CKD-EPI at screening.

According to an aspect of the invention the patient has a decreased renal function with
a glomerular filtration rate (GFR or eGFR) below 75 mL/min/1.73 m², for example determined via CKD-EPI at screening.

According to one embodiment of the methods as described hereinbefore and hereinafter the patient has an eGFR equal to or greater than 20 mL/min/1.73 m².

According to one embodiment of the methods as described hereinbefore and hereinafter the patient has an eGFR equal to or greater than 30 mL/min/1.73 m².

According to another embodiment of the methods as described hereinbefore and hereinafter the patient has an eGFR equal to or greater than 45 mL/min/1.73 m².

According to a variant of this aspect of the invention the patient has an elevated level of proteinuria, for example of equal or greater than 200 mg protein per day (24 h urine protein),

According to a variant of this aspect of the invention the patient has an elevated protein-to-creatinine ratio (PCR) greater or equal than 300 mg/g creatinine.

According to a variant of this aspect of the invention the patient has an elevated albumin-to-creatinine ratio (ACR) greater or equal than 200 mg/g creatinine.

According to a variant of this aspect of the invention the patient has a PCR smaller or equal than 10.0 g/g creatinine.

According to an embodiment of the invention the patient is an adult patient.

According to an aspect of the present invention the patient in need thereof displays impaired production, deposition or function of the collagen IV alpha345 network, for example compared to a normal control patient.

According to a further aspect of the present invention the patient in need thereof displays an impaired or reduced expression of COL4A5, for example compared to a normal control patient. COL4A5 encodes the collagen IV alpha5 chain.

According to a yet further aspect of the present invention the patient in need thereof displays an impaired or reduced expression of COL4A3, for example compared to a normal control patient. COL4A3 is located on chromosome 2 and encodes the collagen IV alpha3 chain.

According to a yet further aspect of the present invention the patient in need thereof displays an impaired or reduced expression of COL4A4, for example compared to a normal control patient. COL4A4 is located on chromosome 2 and encodes the collagen IV alpha4 chain.

According to a yet further aspect of the present invention the patient in need thereof displays an impaired or reduced expression of COL4A3 and COL4A4, for example compared to a normal control patient.

According to another aspect of the present invention the patient in need thereof carries one or more genetic mutations in COL4A5.

According to another aspect of the present invention the patient in need thereof carries one or more genetic mutations in COL4A3.

According to another aspect of the present invention the patient in need thereof carries one or more genetic mutations in COL4A4.

According to another aspect of the present invention the patient in need thereof carries one or more genetic mutations in COL4A3 and COL4A4.

According to an aspect of the present invention the patient is diagnosed as having Alport Syndrome. According to a variant of this aspect the patient is diagnosed as having X-linked Alport Syndrome. According to another variant of this aspect the patient is diagnosed as having autosomal dominant or autosomal recessive Alport Syndrome.

According to an embodiment of the methods as described hereinbefore and hereinafter empagliflozin is administered at a dose in a range from 0.5 to 25 mg per day, in particular 2.5 to 25 mg per day, for example at a dose of 0.5 mg, 1 mg, 2.5 mg, 5 mg, 7.5 mg, 10 mg or 25 mg per day to the patient. The administration of empagliflozin may occur one or two times a day, most preferably once a day. For example a dose for once daily administration is 2.5 mg, 5 mg, 10 mg or 25 mg. The preferred route of administration is oral administration.

According to a particular aspect of the present invention empagliflozin is administered at a dose of 2.5 mg per day to the patient.

According to a particular aspect of the present invention empagliflozin is administered at a dose of 5 mg per day to the patient.

According to a particular aspect of the present invention empagliflozin is administered at a dose of 10 mg per day to the patient.

According to another particular aspect of the present invention empagliflozin is administered at a dose of 25 mg per day to the patient.

Preferably empagliflozin is administered orally to the patient once daily.

Thus according to an embodiment the pharmaceutical compositions or dosage forms according to the invention comprise empagliflozin in an amount from 0.5 to 25 mg, in particular 2.5 to 25 mg, for example 0.5 mg, 1 mg, 2.5 mg, 5 mg, 7.5 mg, 10 mg or 25 mg. For example a solid pharmaceutical composition or dosage form accoding to the invention comprises 2.5 mg, 5 mg, 10 mg or 25 mg empagliflozin.

In one embodiment, patients within the meaning of this invention may include patients with Alport syndrome who have not previously been treated with a drug to treat Alport syndrome. Thus, in an embodiment, the therapies described herein may be used in Alport-syndrome-drug-naïve patients.

Furthermore, it can be found that the administration of a pharmaceutical composition according to this invention results in no risk or in a low risk of hypoglycemia. Therefore, a treatment or prophylaxis according to this invention is also advantageously possible in those patients showing or having an increased risk for hypoglycemia.

By the administration of empagliflozin excessive blood glucose is excreted through the urine of the patient based on the SGLT2 inhibiting activity, so that no gain in weight or even a reduction in body weight of the patient may result. Therefore, the methods according to this invention are advantageously suitable in those patients with Alport syndrome who are diagnosed of one or more of the conditions selected from the group consisting of overweight and obesity, in particular class I obesity, class II obesity, class III obesity, visceral obesity and abdominal obesity. In addition a method according to this invention is advantageously suitable in those patients in which a weight increase is contraindicated.

Furthermore empagliflozin is expected to lower blood pressure. Therefore empagliflozin is advantageously suitable in those patients with Alport syndrome in which a blood pressure reduction is indicated.

Moreover empagliflozin is expected to increase hematocrit and hemoglobin levels. Therefore empagliflozin is advantageously suitable in those patients with Alport syndrome in which an increase of hematocrit and hemoglobin levels is indicated.

When this invention refers to patients requiring treatment or prevention, it relates primarily to treatment and prevention in humans. In the scope of this invention patients may be adult patients, adolescents or children. Adult patients are of the age of 18 years or older. Also in the scope of this invention, adolescents are of age 10 to 17 years, preferably of age 13 to 17 years. Also in the scope of this invention, children are of age 2 to 10 years, preferably of age 6 to 10 years.

According to an embodiment of the present invention empagliflozin is administered in combination with one or more other therapeutic substances to the patient. The combined administration may be simultaneously, separately or sequentially.

In one aspect of this embodiment of the present invention, the one or more other therapeutic substances are selected from active substances that are indicated in the treatment or alleviation of symptoms of Alport syndrom, in particular substances to treat albuminuria, proteinuria, renal impairment and/or high blood pressure.

For example empagliflozin is adminstered in combination with one or more active substances selected from the group consisting of angiotensin receptor blockers (ARB), angiotensin-converting enzyme (ACE) inhibitors, beta-blockers, aldosterone antagonists, diuretics, angiotensin receptor-neprilysin inhibitor (ARNi), calcium channel blockers, mineralcorticoid receptor antagonists and ivabradine.

Furthermore the present invention also relates to a pharmaceutical composition or pharmaceutical dosage form, for example a tablet or a capsule, comprising empagliflozin and at least one pharmaceutically active ingredient selected from the group consisting of angiotensin receptor blockers (ARB), angiotensin-converting enzyme (ACE) inhibitors, beta-blockers, aldosterone antagonists, diuretics, angiotensin receptor-neprilysin inhibitor (ARNi), calcium channel blockers, mineralcorticoid receptor antagonists and ivabradine.

Examples of angiotensin II receptor blockers (ARBs) are telmisartan, candesartan, valsartan, losartan, irbesartan, olmesartan, azilsartan and eprosartan; the dosage(s) of some of these medications are for example shown below:
- Candesartan (Atacand), 4 mg, 8 mg, 16 mg, or 32 mg of candesartan cilexetil
- Eprosartan (Teveten), 400 mg or 600 mg
- Irbesartan (Avapro), 75 mg, 150mg, or 300 mg of irbesartan.
- Losartan (Cozaar), 25 mg, 50 mg or 100 mg of losartan potassium
- Telmisartan (Micardis) , 40 mg or 80 mg
- Telmisartan (Micardis HCT) , 40 mg/12.5 mg, 80 mg/12.5 mg, and 80 mg/25 mg each of telmisartan and hydrochlorothiazide
- Telmisartan/amlodipine (Twynsta) , 40 mg/5 mg, 40 mg/10 mg, 80 mg/5 mg and 80 mg/ 10 mg each of telmisartan and amlodipine
- Valsartan (Diovan) , 40 mg, 80 mg, 160 mg or 320 mg of valsartan

Examples of Angiotensin-Converting Enzyme (ACE) inhibitors are benazepril, captopril, ramipril, lisinopril, Moexipril, cilazapril, quinapril, captopril, enalapril, benazepril, perindopril, fosinopril and trandolapril; the dosage(s) of some of these medications are for example shown below:
- Benazepril (Lotensin), 5 mg, 10 mg, 20 mg, and 40 mg for oral administration
- Captopril (Capoten), 12.5 mg, 25 mg, 50 mg, and 100 mg as scored tablets for oral administration
- Enalapril (Vasotec), 2.5 mg, 5 mg, 10 mg, and 20 mg tablets for oral administration
- Fosinopril (Monopril), for oral administration as 10 mg, 20 mg, and 40 mg tablets
- Lisinopril (Prinivil, Zestril), 5 mg, 10 mg, and 20 mg tablets for oral administration
- Moexipril (Univasc), 7.5 mg and 15 mg for oral administration
- Perindopril (Aceon), 2 mg, 4 mg and 8 mg strengths for oral administration
- Quinapril (Accupril), 5 mg, 10 mg, 20 mg, or 40 mg of quinapril for oral administration
- Ramipril (Altace), 1.25 mg, 2.5 mg, 5, mg, 10 mg
- Trandolapril (Mavik) , 1 mg, 2 mg, or 4 mg of trandolapril for oral administration

Examples of beta-blockers are acebutolol, atenolol, betaxolol, bisoprolol, celiprolol, metoprolol, nebivolol, propranolol, timolol and carvedilol; the dosage(s) of some of these medications are for example shown below:
- Acebutolol (Sectral), 200 or 400 mg of acebutolol as the hydrochloride salt
- Atenolol (Tenormin), 25, 50 and 100 mg tablets for oral administration
- Betaxolol (Kerlone), 10-mg and 20-mg tablets for oral administration
- Bisoprolol/hydrochlorothiazide (Ziac), 2.5/6 mg, 5/6.25 mg, 10/6.25 mg
- Bisoprolol (Zebeta), 5 and 10 mg tablets for oral administration
- Metoprolol (Lopressor, Toprol XL), 50- and 100-mg tablets for oral administration and in 5-mL ampuls for intravenous administration
- Propranolol (Inderal), 10 mg, 20 mg, 40 mg, 60 mg, and 80 mg tablets for oral administration
- Timolol (Blocadren), 5 mg, 10 mg or 20 mg timolol maleate for oral administration.

Examples of aldosterone antagonists are spironolactone, eplerenone, canrenone and fineronone; the dosage(s) of some of these medications are for example shown below:
- spironolactone (e.g. Aldactone), 25 or 50 mg once daily or every second day,
- eplerenone (e.g. Inspra), 25 or 50 mg once daily.

Examples of diuretics are bumetanide, hydrochlorothiazide, chlortalidon, chlorothiazide, hydrochlorothiazide, xipamide, indapamide, furosemide, piretanide, torasemide, spironolactone, eplerenone, amiloride and triamterene; for example these medications are thiazide diuretics, e.g. chlorthalidone, HCT, loop diuretics, e.g. furosemide, torasemide or potassium-sparing diuretics, e.g. eplerenone, or combination thereof; the dosage(s) of some of these medications are for example shown below:
- Amiloride (Midamor), 5 mg of anhydrous amiloride HCl
- Bumetanide (Bumex), available as scored tablets, 0.5 mg (light green), 1 mg (yellow) and 2 mg (peach) for oral administration
- Chlorothiazide (Diuril),
- Chlorthalidone (Hygroton)
- Furosemide (Lasix)
- Hydro-chlorothiazide (Esidrix, Hydrodiuril)
- Indapamide (Lozol) and Spironolactone (Aldactone)
- Eplerenone (Inspra)

An example of an angiotensin receptor-neprilysin inhibitor (ARNi) is a combination of valsartan and sacubitril (Entresto).

Examples of calcium channel blockers are amlodipine, nifedipine, nitrendipine, nisoldipine, nicardipine, felodipine, lacidipine, lercanipidine, manidipine, isradipine, nilvadipine, verapamil, gallopamil and diltiazem.

Examples of medications that lower blood pressure include angiotensin II receptor blockers (ARBs), Angiotensin-Converting Enzyme (ACE) inhibitors, beta-blockers, diuretics and calcium channel blockers.

In one aspect of this embodiment, the number, dosage and/or regimen of said medications or other therapeutic substances to treat or alliviate symptoms of Alport syndrom is reduced in said patient, while the administration of empagliflozin is initiated and/or continued.

Examples of active substances in the above described groups are known to the one skilled in the art, including their dose strengths, administration schemes and formulations.

Within this invention it is to be understood that the combinations, compositions or administrations in combination according to this invention may envisage the simultaneous, sequential or separate administration of the active components or ingredients.

In this context, "combination" or "combined" within the meaning of this invention may include, without being limited, fixed and non-fixed (e.g. free) forms (including kits) and uses, such as e.g. the simultaneous, sequential or separate use of the components or ingredients.

The combined administration of this invention may take place by administering the active components or ingredients together, such as e.g. by administering them simultaneously in one single or in two separate formulations or dosage forms. Alternatively, the administration may take place by administering the active components or ingredients sequentially, such as e.g. successively in two separate formulations or dosage forms.

For the combination therapy of this invention the active components or ingredients may be administered separately (which implies that they are formulated separately) or formulated altogether (which implies that they are formulated in the same preparation or in the same dosage form). Hence, the administration of one element of the combination of the present invention may be prior to, concurrent to, or subsequent to the administration of the other element of the combination.

Unless otherwise noted, combination therapy may refer to first line, second line or third line therapy, or initial or add-on combination therapy or replacement therapy.

The methods according to this invention are particularly suitable in the long term treatment or prophylaxis of the diseases and/or conditions as described hereinbefore and hereinafter. The term "long term" as used hereinbefore and hereinafter indicates a treatment of or administration in a patient within a period of time longer than 12 weeks, preferably longer than 25 weeks, even more preferably longer than 1 year.

The pharmaceutical composition comprising empagliflozin according to the invention may be formulated for oral or parenteral (including intramuscular, sub-cutaneous and intravenous) administration in liquid or solid form or in a form suitable for administration by inhalation or insufflation. Oral administration is preferred. The pharmaceutical composition may be formulated in the form of tablets, granules, fine granules, powders, capsules, caplets, soft capsules, pills, oral solutions, syrups, dry syrups, chewable tablets, troches, effervescent tablets, drops, suspension, fast dissolving tablets, oral fast-dispersing tablets, etc.. The pharmaceutical composition and the dosage forms preferably comprises one or more pharmaceutical acceptable carriers which must be "acceptable" in the sense of being compatible with the other ingredients of the formulation and not deleterious to the recipient thereof. Examples of pharmaceutically acceptable carriers are known to the one skilled in the art. Preferred pharmaceutical compositions are described for example in WO 2010/092126.

The pharmaceutical compositions and methods according to this invention show advantageous effects in the treatment and prevention of those diseases and conditions as described hereinbefore. Further advantageous effects may be seen for example with respect to efficacy, dosage strength, dosage frequency, pharmacodynamic properties, pharmacokinetic properties, fewer adverse effects, convenience, compliance, etc..

Methods for the manufacture of empagliflozin are known to the one skilled in the art. Advantageously, empagliflozin according to this invention can be prepared using synthetic methods as described in the literature, including patent applications as cited hereinbefore. Preferred methods of manufacture are described in the WO 2006/120208 and WO 2007/031548. With regard to empagliflozin an advantageous crystalline form is described in the international patent application WO 2006/117359.

Further embodiments, features and advantages of the present invention may become apparent from the following examples. The following examples serve to illustrate, by way of example, the principles of the invention without restricting it.

### Examples

### Example 1: Treatment of patients with Alport syndrome

The longterm impact on the renal function and other parameters of treatment with empagliflozin in a relevant population of patients with Alport syndrome is investigated as follows:
Empagliflozin is administered to patients in a randomised, double-blind, placebo controlled, parallel group study to compare treatment with empagliflozin, for example 10 mg once daily, with placebo as add-on therapy to standard of care in patients with Alport syndrome. The duration of the patients is preferably a long term treatment, for example 24, 48, 50, 52, 102 or 104 weeks, which may include a post-treatment follow-up period of for example 2 weeks.

Diagnosis of Alport's syndrome (hereditary nephritis) includes a family history of renal failure and deafness suggestive for Alport's syndrome, or skin or renal biopsy-proven diagnosis, or diagnosis by molecular genetic testing.

Patients, in particular adults, include individuals with Alport syndrome and presence of chronic kidney disease defined by:
- eGFR <75 ml/min/1.73m², for example determined via CKD-EPI at screening, and
- high level of proteinuria defined as urinary protein-creatinine ratio (PCR) ≥ 300 mg/g creatinine or very high levels of albuminuria, i.e. macroalbuminuria, defined as UACR ≥ 200 mg/g creatinine, in particular defined as UACR ≥ 300 mg/g creatinine.
   **OR**
- eGFR <90 ml/min/1.73m², for example determined via CKD-EPI at screening, and
- very high levels of albuminuria, defined as UACR ≥ 500 mg/g creatinine.

Furthermore patients, in particular adolescents (e.g. aged 10 to 17 years) or children (e.g. aged 2-10 or 6-10 years), include individuals with Alport syndrome and presence of chronic kidney disease defined by:
- high level of albuminuria defined as UACR ≥ 300mg/g creatinine.

In particular said PCR has to be present at screening and at least on one additional, documented occasion within 12 month prior to screening.

Patients preferably have a renal function greater or equal than 20 ml/min/1.73m². Furthermore patients preferably have a PCR smaller or equal than 10.0 g/g.

Furthermore patients include those with stable single RAS blockade background therapy, for example either ACE-inhibitor or ARB with unchanged daily dose.

Patients are adult patients. Furthermore the study may include adolescent patients (e.g. aged 10 to 18 years) and/or children (e.g. aged 2-10 or 6-10 years).

The primary endpoint of the study is change from baseline in eGFR, assessed for example by CKD-EPI.

The key secondary endpoint relates to change from baseline in urinary albumin-to-creatinine ratio (UACR, mg/g creatinine), for example the time-weighted average of percentage change from baseline.

Further secondary endpoints are
- Change from baseline in eGFR
- Change from baseline in UACR
- Change from baseline in PCR
- Regression or progression in UACR category
- Change from baseline in weight and blood pressure.

Further endpoints may relate to
- Slope analysis for renal function over time
- incidence of rapid renal function decline, for example annual loss greater or equal than 5 ml/min/year
- Change from baseline in metabolic parameters (e.g. FPG, lipids, uric acid).

### Example 2: Pharmaceutical Composition and Dosage Form

The following example of solid pharmaceutical compositions and dosage forms for oral administration serves to illustrate the present invention more fully without restricting it to the contents of the example. Further examples of compositions and dosage forms for oral administration, are described in WO 2010/092126. The term "active substance" denotes empagliflozin according to this invention, especially its crystalline form as described in WO 2006/117359 and WO 2011/039107.

Tablets containing 2.5 mg, 5 mg, 10 mg or 25 mg of the active substance empagliflozin.

Amounts of the ingredients are provided in mg per film-coated tablet.

| **Active substance** | 2.5 mg/ per tablet | 5 mg/ per tablet | 10 mg/ per tablet | 25 mg/ per tablet |
|---|---|---|---|---|
| **Wet granulation** | | | | |
| Empagliflozin | 2.5000 | 5.000 | 10.00 | 25.00 |
| Lactose Monohydrate | 40.6250 | 81.250 | 162.50 | 113.00 |
| Microcrystalline Cellulose | 12.5000 | 25.000 | 50.00 | 40.00 |
| Hydroxypropyl Cellulose | 1.8750 | 3.750 | 7.50 | 6.00 |
| Croscarmellose Sodium | 1.2500 | 2.500 | 5.00 | 4.00 |
| Purified Water | q.s. | q.s. | q.s. | q.s. |

| **Dry Adds** | | | | |
|---|---|---|---|---|
| Microcrystalline Cellulose | 3.1250 | 6.250 | 12.50 | 10.00 |
| Colloidal silicon dioxide | 0.3125 | 0.625 | 1.25 | 1.00 |
| Magnesium stearate | 0.3125 | 0.625 | 1.25 | 1.00 |
| Total core | 62.5000 | 125.000 | 250.00 | 200.00 |

| **Film Coating** | | | | |
|---|---|---|---|---|
| Film coating system | 2.5000 | 4.000 | 7.00 | 6.00 |
| Purified Water | q.s. | q.s. | q.s. | q.s. |
| **Total** | **65.000** | **129.000** | **257.00** | **206.00** |

Details regarding the manufacture of the tablets, the active pharmaceutical ingredient, the excipients and the film coating system are described in WO 2010/092126.

## Claims

1. Empagliflozin for use in treating Alport syndrome in a patient in need thereof.

2. Empagliflozin for use according to claim 1 in delaying progression of chronic kidney disease in a patient diagnosed with Alport syndrome.

3. Empagliflozin for use according to claim 1 in treating or alleviating glomerular hypertension in a patient diagnosed with Alport syndrome.

4. Empagliflozin for use according to claim 1 in treating, including slowing or reversing the progression of albuminuria and/or proteinuria in a patient diagnosed with Alport syndrome.

5. Empagliflozin for use according to one or more of the claims 1 to 4 wherein the patient has an eGFR equal to or greater than 20 mL/min/1.73m².

6. Empagliflozin for use according to one or more of the claims 1 to 5 wherein the adult patient has an eGFR lower than 75 mL/min/1.73m² or lower than 90 mL/min/1.73m².

7. Empagliflozin for use according to one or more of the claims 1 to 6 wherein the patient has an elevated protein-to-creatinine ratio (PCR) greater or equal than 300 mg/g creatinine, an elevated level of proteinuria of equal or greater than 200 mg protein per day (24 h urine protein) or an elevated albumin-to-creatinine ratio (ACR) greater or equal than 200 mg/g creatinine or greater or equal than 300 mg/g creatinine.

8. Empagliflozin for use according to one or more of the claims 1 to 7 wherein empagliflozin is administered at a dose in a range from 1 mg to 25 mg per day to the patient.

9. Empagliflozin for use according to claim 8 wherein empagliflozin is administered at a dose of 10 mg per day to the patient.

10. Empagliflozin for use according to one or more of the claims 1 to 9 wherein empagliflozin is administered in combination with one or more other therapeutic substances to the patient.

11. Empagliflozin for use according to claim 10 wherein the one or more other therapeutic substances are selected from the group consisting of angiotensin receptor blockers (ARB), angiotensin-converting enzyme (ACE) inhibitors, beta-blockers, aldosterone antagonists, diuretics, angiotensin receptor-neprilysin inhibitor (ARNi), calcium channel blockers, mineralcorticoid receptor antagonists and ivabradine.

12. A pharmaceutical composition comprising empagliflozin for use as defined in one or more of the claims 1 to 11.

13. The pharmaceutical composition for use according to claim 12 comprising empagliflozin in combination with one or more other therapeutic substances selected from the group consisting of angiotensin receptor blockers (ARB), angiotensin-converting enzyme (ACE) inhibitors, beta-blockers, aldosterone antagonists, diuretics, angiotensin receptor-neprilysin inhibitor (ARNi), calcium channel blockers, mineralcorticoid receptor antagonists and ivabradine.

14. The pharmaceutical composition for use according to claim 12 or 13 comprising an amount of 1 to 25 mg of empagliflozin.

## Patentansprüche

1. Empagliflozin zur Verwendung bei der Behandlung des Alport-Syndroms bei einem Patienten, der dies benötigt.

2. Empagliflozin zur Verwendung gemäß Anspruch 1 zur Verzögerung des Fortschreitens einer chronischen Nierenerkrankung bei einem Patienten, bei dem ein Alport-Syndrom diagnostiziert wurde.

3. Empagliflozin zur Verwendung gemäß Anspruch 1 bei der Behandlung oder Linderung von glomerulärem Bluthochdruck bei einem Patienten, bei dem ein Alport-Syndrom diagnostiziert wurde.

4. Empagliflozin zur Verwendung gemäß Anspruch 1 bei der Behandlung, einschließlich der Verlangsamung oder Umkehrung des Fortschreitens von Albuminurie und/oder Proteinurie bei einem Patienten, bei dem ein Alport-Syndrom diagnostiziert wurde.

5. Empagliflozin zur Verwendung gemäß einem oder mehreren der Ansprüche 1 bis 4, wobei der Patient eine eGFR gleich oder größer als 20 ml/min/1,73m² hat.

6. Empagliflozin zur Verwendung gemäß einem oder mehreren der Ansprüche 1 bis 5, wobei der erwachsene Patient eine eGFR von weniger als 75 mL/min/1,73m² oder weniger als 90 mL/min/1,73m² aufweist.

7. Empagliflozin zur Verwendung gemäß einem oder mehreren der Ansprüche 1 bis 6, wobei der Patient ein erhöhtes Protein-Kreatinin-Verhältnis (PCR) von größer oder gleich 300 mg/g Kreatinin, eine erhöhte Proteinurie von gleich oder größer als 200 mg Protein pro Tag (24-Stunden-Urinprotein) oder ein erhöhtes Albumin-Kreatinin-Verhältnis (ACR) von größer oder gleich 200 mg/g Kreatinin oder größer oder gleich 300 mg/g Kreatinin aufweist.

8. Empagliflozin zur Verwendung gemäß einem oder mehreren der Ansprüche 1 bis 7, wobei Empagliflozin in einer Dosis im Bereich von 1 mg bis 25 mg pro Tag an den Patienten verabreicht wird.

9. Empagliflozin zur Verwendung gemäß Anspruch 8, wobei Empagliflozin in einer Dosis von 10 mg pro Tag an den Patienten verabreicht wird.

10. Empagliflozin zur Verwendung gemäß einem oder mehreren der Ansprüche 1 bis 9, wobei Empagliflozin in Kombination mit einer oder mehreren anderen therapeutischen Substanzen an den Patienten verabreicht wird.

11. Empagliflozin zur Verwendung gemäß Anspruch 10, wobei die eine oder die mehreren anderen therapeutischen Substanzen ausgewählt sind aus der Gruppe bestehend aus Angiotensinrezeptorblockern (ARB), Angiotensin-Converting-Enzyme (ACE)-Inhibitoren, Betablockern, Aldosteronantagonisten, Diuretika, Angiotensinrezeptor-Neprilysin-Inhibitor (ARNi), Kalziumkanalblockern, Mineralkortikoidrezeptorantagonisten und Ivabradin.

12. Pharmazeutische Zusammensetzung, umfassend Empagliflozin zur Verwendung wie in einem oder mehreren der Ansprüche 1 bis 11 definiert.

13. Pharmazeutische Zusammensetzung zur Verwendung gemäß Anspruch 12, umfassend Empagliflozin in Kombination mit einer oder mehreren anderen therapeutischen Substanzen, ausgewählt aus der Gruppe, bestehend aus Angiotensinrezeptorblockern (ARB), Angiotensin-Converting-Enzyme (ACE)-Inhibitoren, Betablockern, Aldosteronantagonisten, Diuretika, Angiotensinrezeptor-Neprilysin-Inhibitor (ARNi), Calciumkanalblockern, Mineralcorticoidrezeptorantagonisten und Ivabradin.

14. Pharmazeutische Zusammensetzung zur Verwendung gemäß Anspruch 12 oder 13, umfassend eine Menge von 1 bis 25 mg Empagliflozin.

## Revendications

1. Empagliflozine pour son utilisation dans le traitement du syndrome d'Alport chez un patient le nécessitant.

2. Empagliflozine pour son utilisation selon la revendication 1 dans le retardement de la progression d'une maladie rénale chronique chez un patient diagnostiqué avec le syndrome d'Alport.

3. Empagliflozine pour son utilisation selon la revendication 1 dans le traitement ou le soulagement d'une hypertension glomérulaire chez un patient diagnostiqué avec le syndrome d'Alport.

4. Empagliflozine pour son utilisation selon la revendication 1 dans le traitement, incluant le ralentissement ou l'inversion de la progression d'une albuminurie et/ou d'une protéinurie chez un patient diagnostiqué avec le syndrome d'Alport.

5. Empagliflozine pour son utilisation selon une ou plusieurs des revendications 1 à 4 dans laquelle le patient présente un eGFR égal ou supérieur à 20 mL/min/1,73 m².

6. Empagliflozine pour son utilisation selon une ou plusieurs des revendications 1 à 5 dans laquelle le patient adulte présente un eGFR inférieur à 75 mL/min/1,73 m² ou inférieur à 90 mL/min/1,73 m².

7. Empagliflozine pour son utilisation selon une ou plusieurs des revendications 1 à 6 dans laquelle le patient présente un rapport protéines / créatinine (PCR) élevé supérieur ou égal à 300 mg/g de créatinine, un niveau élevé de protéinurie égal ou supérieur à 200 mg de protéines par jour (protéines d'urine en 24 h) ou un rapport albumine / créatinine (ACR) élevé supérieur ou égal à 200 mg/g de créatinine ou supérieur ou égal à 300 mg/g de créatinine.

8. Empagliflozine pour son utilisation selon une ou plusieurs des revendications 1 à 7 dans laquelle l'empagliflozine est administrée à une dose se trouvant dans une plage de 1 mg à 25 mg par jour au patient.

9. Empagliflozine pour son utilisation selon la revendication 8 dans laquelle l'empagliflozine est administrée à une dose de 10 mg par jour au patient.

10. Empagliflozine pour son utilisation selon une ou plusieurs des revendications 1 à 9 dans laquelle l'empagliflozine est administrée en combinaison avec une ou plusieurs autres substances thérapeutiques au patient.

11. Empagliflozine pour son utilisation selon la revendication 10 dans laquelle la une ou les plusieurs autres substances thérapeutiques sont choisies dans le groupe consistant en bloqueurs de récepteur d'angiotensine (ARB), inhibiteurs d'enzyme de conversion d'angiotensine (ACE), bêta-bloquants, antagonistes d'aldostérone, diurétiques, inhibiteur de récepteur d'angiotensine-néprilysine (ARNi), bloqueurs de canaux calciques, antagonistes de récepteur de minéralocorticoïde et ivabradine.

12. Composition pharmaceutique comprenant une empagliflozine pour son utilisation tel que défini dans une ou plusieurs des revendications 1 à 11.

13. Composition pharmaceutique pour son utilisation selon la revendication 12 comprenant une empagliflozine en combinaison avec une ou plusieurs autres substances thérapeutiques choisies dans le groupe consistant en bloqueurs de récepteur d'angiotensine (ARB), inhibiteurs d'enzyme de conversion d'angiotensine (ACE), bêta-bloquants, antagonistes d'aldostérone, diurétiques, inhibiteur de récepteur d'angiotensine-néprilysine (ARNi), bloqueurs de canaux calciques, antagonistes de récepteur de minéralocorticoïde et ivabradine.

14. Composition pharmaceutique pour son utilisation selon la revendication 12 ou 13 comprenant une quantité de 1 mg à 25 mg d'empagliflozine.
